# EUROPEAN PATENT SPECIFICATION

(11) **EP 0 564 735 B1**
(45) Date of publication and mention of the grant of the patent: **18.11.1998**
(21) Application number: 92303259.3
(22) Date of filing: 10.04.1992
(51) Int. Cl.: G01N 33/569, G01N 15/14, G01N 33/58

(54) **Method for detection of CMV infection**
Verfahren zum Nachweis einer CMV-Infektion
Méthode de détection d'une infection CMV

(43) Date of publication of application: 13.10.1993
(73) Proprietor: Totterman, Thomas, S-752 27 Uppsala (SE); Hanas, Erland, S-756 55 Uppsala (SE)
(72) Inventor: Totterman, Thomas, S-752 27 Uppsala (SE); Hanas, Erland, S-756 55 Uppsala (SE)
(74) Representative: Ruffles, Graham Keith

(56) References cited:
- EP-A- 257 559
- EP-A- 336 379
- CLINICAL IMMUNOLOGY AND IMMUNOPATHOLOGY vol. 51, 1989, NEW YORK US pages 157 - 171 D.L.SIEGEL ET AL. 'Discriminating rejection from CMV infection in renal allograft recipients using flow cytometry'
- TRANSPLANTATION vol. 47, no. 5, May 1989, BALTIMORE pages 817 - 823 T.H.T TTERMAN ET AL. 'Immunologic diagnosis of kidney rejection using FACS analysis of graft-infiltrating functional and activated T and NK cell subsets'
- TRANSPLANTATION vol. 45, no. 1, January 1988, NEW YORK US pages 132 - 138 A.M. M.KOOTTE ET AL. 'Enumeration of leu2a+, leu2a+-dr+, and leu2a+-leu15+ cells in peripheral blood of renal transplant patients'
- ARCHIVES OF PATHOLOGY & LABORATORY MEDICINE vol. 116, no. 1, January 1992, CHICAGO US D.A.SCHWARTZ ET AL. 'Characterization of the fetal inflammatory response to cytomegalovirus placentitis'

## Description

### Field of the Invention

This invention relates to a method for the detection of cytomegalovirus infection in solid organ transplant patients, and more specifically relates to an immunophenotyping method for the detection of cytomegalovirus infection in renal transplant patients.

### Background of the Invention

Cytomegalovirus (CMV) is a ubiquitous exogenous DNA virus which incorporates into the human genome. It is the most frequent and important viral pathogen complicating organ transplantation. Its manifestations include a variety of syndromes such as hepatitis, pneumonitis, colitis and retinitis. Prolonged fever, malaises, fatigue, night sweats, arthralgia and myalgias are prodromes. Liver function abnormalities, leukopenia, thrombocytopenia and atypical lymphocytosis also may be observed. Graft dysfunction, as manifested by an increase in serum creatinine, can be caused by direct viral cytopathic effect and must be differentiated from true graft refection because the former does not respond well to increased immunosuppressive therapy.

CMV infection can be due to either primary infection, reactivation of dormant endogenous infection or reinfection with a new CMV strain. The reported incidence of CMV infection in renal allograft recipients receiving prednisolone and azathioprine ("Pred-Aza") or prednisolone and cyclosporine A ("Pred-CsA") as an immunosuppressive therapy varies between 50% and 70%. CMV infection is frequently asymptomatic, and clinically overt disease occurs in less than half of infected patients on Pred-Aza therapy. (Clinically overt disease is defined for these purposes as the combination of serologic and/or culture diagnosis of CMV infection associated with fever of greater than 38°C and one or more of the following clinical syndromes: pneumonitis, confusion, gastrointestinal bleeding or pancreatitis. If none of these clinical signs is present, at least two of the following three criteria must be met: histologic signs of CMV; white blood cell count of less than 5,000/mm³; abnormal liver function; or a 4X rise to at least 35 Units of STK.) In 516 renal recipients on Pred-CsA therapy, clinically overt disease developed in 31% of patients with verified infection. This represented 8.7% of the transplanted population.

The laboratory and clinical diagnosis of CMV infection are both unsatisfactory at the present time. Virus cultures, CMV, specific antibodies, monoclonal antibodies against viral replication genes and the polymerase chain reaction ("PCR") are laboratory methods with different limitations for diagnosis of CMV infection. Traditional virus isolations are time-consuming (4-6 weeks). The rapid immunofluoresence technique (1-2 days) for detection of early CMV antigens in susceptible cultured target cells, such as human foreskin fibroblasts, is in practice frequently positive also in asymptomatic cases. Monoclonal antibodies can be used for the immunocytochemical demonstration of CMV antigens in peripheral blood leukocytes. This is a slow and tedious technique since very few cells are positive. CMV-specific antibody titers measured by Elisa are useful but may be quite unreliable in immunosuppressed subjects. Serum levels of DNA replication-associated enzymes, e.g. thymidine kinase ("STK"), may be of prognostic value but are not virus specific. Finally, PCR involving CMV specific probes has not been clinically evaluated.

A limited number of attempts have been made to correlate changes in circulating lymphocyte phenotype with clinical CMV infection or rejection in kidney allograft transplanted patients. Using 2-color flow cytometry, Siegel et al., Clin. Immunol. Immunopathol., 1989: 51, 157-71, showed that the number of circulating HLA-DR cells expressing CD2 increased during CMV infection, whereas the number of cells expressing CD25 and CD3 increased during rejection. Kootte et al., Transplantation, 1988: 45, 132-38, reported an increase in circulating DR⁺CD8⁺ cells and CD11b⁺CD8⁺ cells during both CMV infection and rejection, whereas Gross et al., Transpl. Proc., 1988: 20, 102-04, observed a decrease in the latter cell type during rejection. Hanas et al., Nephrol. Dial. & Transpl., 1989: 4, 735-39, observed that allograft rejection is associated with a decrease in CD45R⁺CD4⁺ T cells and CD11b⁺CD8⁺ T cells and an increase in HLA-DR⁺CD16⁺ NK cells. These changes occurred both in kidney tissue and in blood. See Hanas et al., supra, and Totterman et al., Transplantation, 1989: 47, 817-23. Taking all of this evidence together, however, no consistent pattern has emerged. Thus, there lacks a method for the detection of CMV infection particularly among transplantation patients.

### Summary of the Invention

The present invention comprises a method for the prediction and detection of CMV infection in solid organ transplant patients. The method comprises the steps of combining a sample of blood or other tissue with at least two fluorescently labelled monoclonal antibodies which recognize the CD45RO and CD45RA antigens on CD4⁺ T cells, counting the number of CD4⁺ T cells that are CD45RA⁺ and CD45RO⁺ and determining the ratio of such cells. An increase in the ratio over time or a ratio of greater than 1 correlates with CMV infection.

More particularly, the present invention provides a method for the predication and detection of CMV infection in solid organ transplant patients comprising the steps of combining a sample of blood or other tissue from the patient with at least two fluorescently labelled monoclonal antibodies which recognise the CD45RO and CD45RA antigens on CD4⁺ T cells, counting on a flow cytometer the number of CD4⁺ T cells that are CD45RA⁺ and CD45RO⁺ and determining the ratio of such cells.

The present invention further provides a method a method for the prediction and detection of CMV infection in solid organ transplant patients comprising the steps of combining a sample of blood or other tissue from the patient with at least three fluorescently labelled monoclonal antibodies which recognise the CD45RO, CD45RA and CD4 antigens on T cells, counting on a flow cytometer number of CD4⁺ T cells that are CD45RA⁺ and CD45RO⁺ and determining the ratio of such cells.

The present invention also includes the use of at least two fluorescently labelled monoclonal antibodies which recognise the CD45RO and CD45RA antigens in the preparation of a kit for use in such a method, and also the use of at least three fluorescently labelled monoclonal antibodies which recognise the CD45RO, CD45RA, and CD4 antigens in the preparation of a kit for use in such a method.

This method is applicable to samples obtained from heart, liver, kidney, lung and other solid organ trabsolant patients. Kidney transplants comprise a preferred embodiment of this invention.

In this method, the cells are counted by means of flow cytometry. Flow cytometry comprises a well known methodology which relies on a flow cytometer and associated computer hardware to identify and count cells as they pass substantially one at a time through a sensing region. In this region, each cell is illuminated by light from single wavelength source, such as an argon or helium/neon laser, and the light scattered in one or more directions as well as the light emitted by the presence of one or more fluorescent dyes is captured and the multiparameter data is recorded and stored in a computer having appropriate software for such purposes. A FACScan brand flow cytometer Becton Dickinson Immunocytometry Systems, "BDIS") tied to an HP310 computer equipped with FACSCan Research Software provides one example of means by which this method can be carried out. For a more detailed description of flow cytometers and flow cytometry, see U.S. Pat. Nos. 4,727,020 and 4,745,285, and see Herzenberg et al., Sci. Amer., 1976: 234, 108-17.

The cells can be fluorescently labelled either by direct conjugation to a fluorescent dye or indirectly by linking an anti-IgG antibody directly to a fluorescent dye. When multiple monoclonal antibodies are used, each of the dyes should have an emission spectra that is distinguishable from the others. It is preferrable that each of the dyes is excitable by light of a single wavelength (e.g., 488nm). Dyes having these properties include fluorescein isothiocyanate ("FITC"), r-phycoerthrin ("PE"), Texas Red (Molecular Probes, Inc.), peridinin chlorophyll complex ("PerCp"), allophycocyanin ("APC") and tandem conjugates thereof. Methods for conjugating such dyes to monoclonal antibodies are well known in the art. One method is described in U.S. Pat. No. 4,520,110. One method for preparing tandem conjugates is described in U.S. Pat. No. 4,542,104.

In a preferred embodiment of this invention, blood from a renal transplant patient is combined with fluorescently labelled CD4, CD45RO and CD45RA monoclonal antibodies and the mixture is analyzed by means of flow cytometry. The ratio of CD4⁺ T cells that are CD45RA⁺ and CD45RO⁺ is then determined. An increasing ratio over time or a ratio of greater than 1 correlates with CMV infection.

### Description of the Drawings

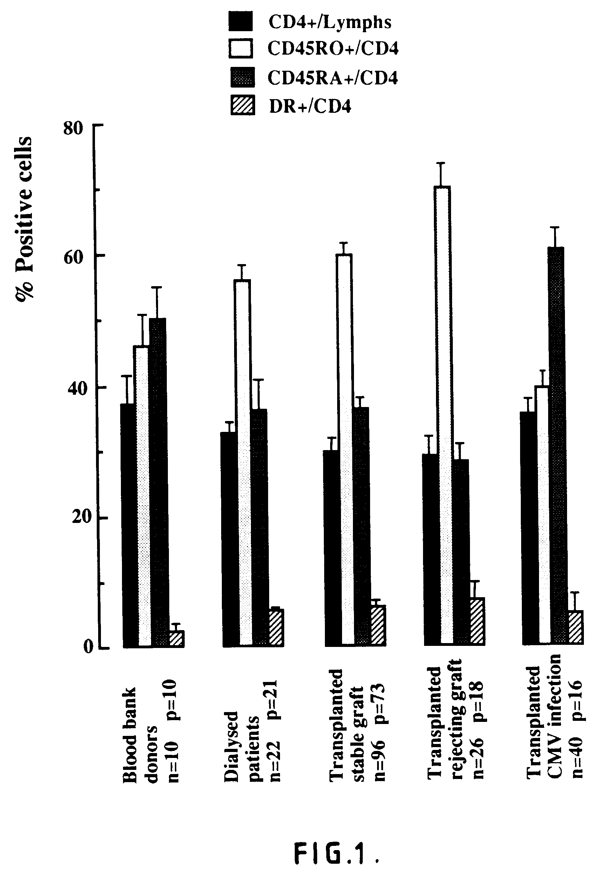

FIG. 1 comprises several histograms of blood samples taken from normal blood bank donors, dialysis patients, stable renal graft patients, renal rejection patients and renal transplant patients with CMV infection and analyzed by flow cytometric 2-color analysis for CD4⁺ cells as a percentage of lymphocytes, CD45RO⁺ cells as a percentage of CD4⁺ cells, CD45RA⁺ cells as a percentage of CD4⁺ cells and HLA-DR⁺ cells as a percentage of CD4⁺ cells.

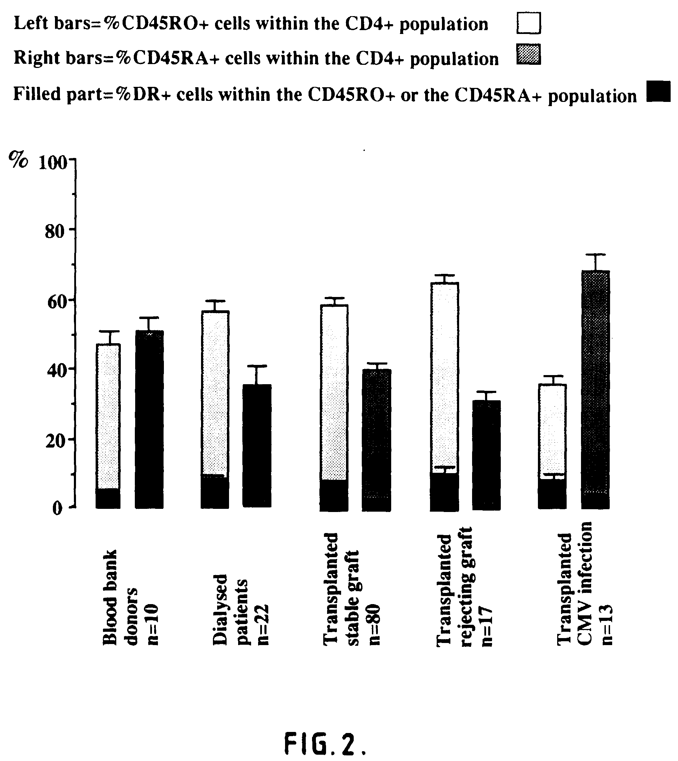

FIG. 2 comprises the same patient groups as in FIG. 1, however, the blood samples were analyzed by 3-color flow cytometric analysis for CD45RO⁺ cells as a percentage of CD4⁺ cells, CD45RA⁺ cells as a percentage of CD4⁺ cells and HLA-DR⁺ cells as a percentage of CD45RO⁺ or CD45RA⁺ cells within the CD4⁺ population.

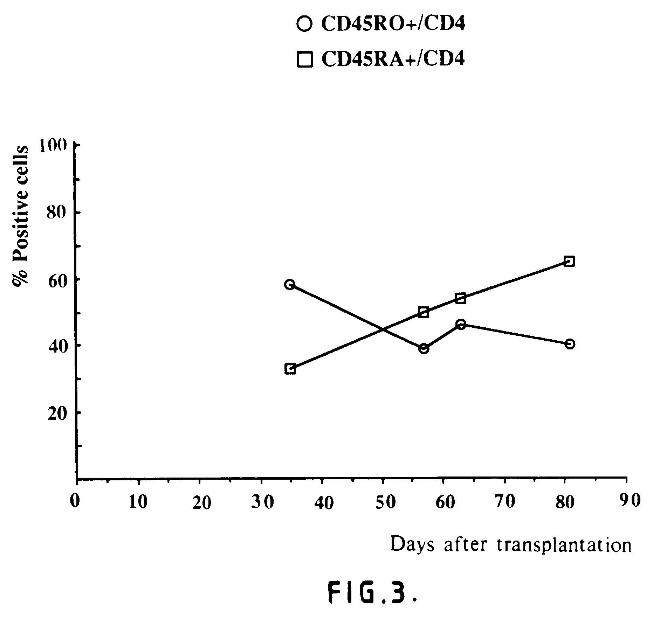

FIG. 3 comprises a plot of the ratio of CD45RA:CD45RO over time in a single patient.

### Detailed Description

The patient population included 21 dialysed uremics as clinical controls and 96 renal allograft recipients admitted to the transplant unit at the University Hospital, Uppsala, Sweden. Ten blood blank donors were included as control subjects without renal disease.

From transplanted patients, a kidney biopsy for histopathology and a peripheral blood sample for flow cytometry analysis were routinely taken according to protocols approved by the Hospital's ethics committee. Biopsy and blood sampling was done during the first week, third week and third month after transplantation or every time a rejection or overt CMV infection was suspected. Retrospectively, each sampling occasion was clinically classified as a situation of stable graft function, graft rejection or overt CMV infection.

The clinical diagnosis of stable graft function was based on unchanged or decreasing serum creatinine levels for 7 days before and 10 days after biopsy. If s-creatinine was elevated more than 10%, the following parameters were assessed to diagnose a rejection episode: histopathology of a kidney biopsy, response to increased immunosuppression, CsA trough levels, s-creatinine changes associated with adjustment of CsA dosage, decreased urinary output, renal scintigraph, absence of urethral obstruction (as judged by ultrasound) and, at times, supplemented by antegrade pyelography, absence of urinary infection and CMV infection.

Diagnosis of CMV infection consisted of: at least a 4X rise in CMV-IgG antibody titer measure by complement fixation or Elisa specific for IgG; seroconversion of CMV negative recipients measured by Elisa specific for IgM; or positive virus culture from blood or bronchoalveolar lavage.

With few exceptions, the patients received basic immunosuppression with CsA (Sandoz), Pred (Aco) and Aza (Wellcome). There were 13 patients who received Pred-Aza only. These subjects where mostly transplanted more than 3 years prior to study and were not found in the CMV infected group. Rejection treatment consisted of solumedrol (Upjohn; 1.25 gm divided on four days) or ATG (Fresenius AG; 3 mg/kg/day for 7-12 days) or ALG (Foundation Merieux, Lyon, France; 5 ml/10 kg/day for 7-10 days) or OKT 3 monoclonal antibody (Ortho Pharmaceuticals; 5 mg/day for 9-11 days).

Ten ml of heparinized peripheral venous blood was collected according to standard aseptic technique and mononuclear cells were isolated on routine density gradients (Ficoll-Hypaque, Pharmacia AB).

For 2-color immunofluoresence staining, blood mononuclear cells were washed and suspended in phosphate-buffered saline ("PBS") with 2% fetal calf serum ("FCS", Gibco) and 0.1% sodium azide. 5 x 10⁵ cells in 25 **m**l were incubated for 10 minutes at room temperature with combinations of FITC and PE conjugated monoclonal antibodies, washed twice and mounted in PBS+2%FCS+1% paraformaldehyde for flow cytometric analysis. The antibodies Leu 3a (CD4), Leu 18 (CD45RO) and anti-HLA-DR (unclustered) are commercially available from BDIS and other commercial sources. The antibody UCHL-1 (CD45RO) was the gift of Dr. P. Beverley, London, and was FITC conjugated before use. This antibody now is commercially available from BDIS.

For 3-color immunofluoresence staining, blood mononuclear cells were extensively washed 3X and suspended in PBS. Careful washing of cells was necessary in order to remove serum and thereby endogenous biotin. 10⁶ cells in 50 ml were incubated for 10 minutes at room temperature with biotin-labelled Leu 3 monoclonal antibody and washed twice in PBS. Cells were thereafter incubated with streptavidin-labelled PE-Texas Red tandem conjugate (Duochrome, BDIS) for 10 minutes and washed once. Finally, cells were incubated with either (i) a combination of FITC-labelled Leu 18 and PE-labelled anti-HLA-DR, washed once and mounted in PBS containing 1% paraformaldehyde before flow cytometric analysis.

Stained cells were analyzed on a FACScan brand flow cytometer (BDIS) equipped with a 15 mW Argon ion laser emitting at 488 nm. Green fluorescence (FITC) was collected through a 530/30 nm bandpass filter, red fluorescence (PE) through a 585/42 filter and SA-TC fluorescence (Texas Red) through a 660 nm long pass filter. Leakage of SA-TC fluorescence into the PE channel was compensated for manually.

Ten-thousand mononuclear cells with lymphocyte scatter properties were gated in order to minimize inclusion of possible monocytes. Five-parameter list mode data was collected and processed with the FACScan Research Software (BDIS). In both instances, forward and side scatter signals were collected in linear mode, while the fluorescence detectors were operated with logarithmic amplification.

Simple group differences were analyzed with the non-parametric Mann-Whitney test. The tests performed assume independence which might be doubtful when more than one sample from each individual was included. To check this effect, estimates were also obtained using the first sample only for each patient. Since this resulted in similar statistical result, data for the total number of samples are given.

Referring to FIG. 1, healthy subjects were compared with either uremic patients on dialysis or renal allograft recipients. There was no statistically significant difference in the percentage of CD4⁺ lymphocytes between patients and healthy controls. In healthy subjects, the proportion of CD45RA⁺ cells dominated over the proportion of CD45RO⁺ cells within the CD4⁺ population. The opposite pattern was observed in kidney patients.

When healthy subjects were compared with transplanted patients having stable grafts, there was an increase in the proportion of CD45RA⁺ cells (46 % 16% mean % 1 S.D. vs. 60 % 18%, p = 0.02), a decrease in CD45RO⁺ cells (51 % 16% vs. 39 % 19%; p = 0.02) and an increase in HLA-DR⁺ cells (2.3 % 1.4% vs. 5.6 % 2.5%; p = 0.002). This overall pattern was similar when comparing both the dialysed group (CD45RO⁺ [46 % 16% vs. 56 % 14%, NS]; CD45RA⁺ [51 % 16% vs. 36 % 18%, NS]; HLA-DR⁺ [2.3 % 1.4% vs. 5.6 % 2.5%, p = 0.0008]) and rejecting graft group (CD45RO⁺ [46 % 16% vs. 68 % 17%, p = 0.004]; CD45RA⁺ [51 % 16% vs. 31 % 15%, p = 0.005]; HLA-DR⁺ [2.3 % 1.4% vs. 7.2 % 11.8%, NS]) with the healthy blood donors. The non-significant ("NS") values might be explained by the smaller number of dialysed and rejecting patients compared to patients with stable grafts.

The picture with respect to transplanted patients exhibiting CMV infection is totally different. During clinically overt CMV infection, the proportion of CD45RO⁺ cells within the CD4⁺ T cell subset was higher compared with rejection (40 % 17% vs. 69 % 18%, p < 0.0001). The opposite was true for CD45RA⁺ cells (61 % 21% vs. 29 % 14%, p < 0.0001). The same pattern was evident when CMV infected patients were compared with stable allograft patients (CD45RO⁺ [40 % 17% vs. 60 % 18%, p < 0.0001]; CD45RA⁺ 61 % 21% vs. 37 % 19%, p < 0.0001] and dialysed patients.

By calculating the ratio between CD45RA⁺ and CD45RO⁺ cells within the CD4⁺ T cell population, it was possible to separate CMV infection from rejection. Thirty four of 40 CMV blood samples had a ratio greater than 1.0, and all but two samples (from one patient) in the rejecting group had a ratio of less than 1.0. (This patient subsequently developed clinical CMV disease.) In the stable graft group (n=96), there were 26 blood samples from 23 patients with a ratio of 1.0 or larger. Only four of these samples were from patients who later developed clinical CMV infection.

Taken together, the CD45RA:CD45RO ratio in patients with rejection, stable grafts and clinical CMV infection were 0.48 % 0.3 (range 0.2 - 1.2), 0.80 % 0.77 (range 0.04 - 5.5) and 1.9 % 1.5 (range 0.2 - 5.4) respectively.

There were no striking differences between these groups. In a previous work, it was reported that the proportions of CD45RA⁺ / CD4⁺ T cells decreased during rejection. In the present study, a decrease was noted which did not reach statistical significance (37 % 19% vs. 29 % 16%, NS). A likely explanation for this is that the former study was done with a different flow cytometer having lower sensitivity. As expected, CD45RO⁺ / CD4⁺ T cells increased in rejecting grafts and was statistically significant (60 % 18% vs. 70 % 18%, p = 0.04).

Referring to FIG. 2, using the 3-color technique, the simultaneous expression of 3 surface markers, i.e. HLA-DR, CD4 and either CD45RA or CD45RO, on lymphocytes was analyzed. The goal was to determine whether HLA-DR was present on CD45RA⁺ or CD45RO⁺ cells. Within all groups of subjects studied, the major HLA-DR expression was found on CD45RO⁺ / CD4⁺ T cells. The HLA-DR expression on CD45RO⁺ / CD4⁺ T cells was increased in patients with renal disease (healthy subjects 3.8 % 1.9% vs. transplanted patients with stable grafts 8.0 % 5.9%, p = 0.02). The HLA-DR expression on CD45RA⁺ CD4⁺ T cells was also increased in uremia (0.9 % 0.5% vs. 3.2 % 4.5%, p = 0.03). The same pattern was true for stable and rejecting allograft recipients compared with blood donors.

In a single study, a 34 year old diabetic man received a kidney from his mother on day 0. (He had not started dialysis before transplantation.) Immunosuppression consisted of Csa, Pred, and Aza. The patient left the hospital on day 15. A rise in s-creatinine was noted on day 21 and because the kidney biopsy showed an interstitial and vascular rejection, solumedrol was instituted. The response was not satisfactory and more solumedrol was given followed by OKT3 therapy. Fever started on day 40 and CMV was isolated from blood and urine on day 50. At the same time, leukopenia, a rise in STK and histological signs of CMV were apparent and natrium foscarnet treatment was initiated. Three weeks later the patient received anti-rejection treatment with ATG. The s-creatinine slowly declined from 437 to 300 **m**M/l.

Three blood samples were taken during this three month period. Referring to FIG. 3, a conversion in the CD45RA/CD45RO ratio was seen between days 35 and 57. Again, this inversion in the ratio correlates with the onset of CMV infection.

The present studies demonstrate that 2 and 3-color flow cytometric analysis of circulating T cell immunophenotypes is of considerable clinical value in identifying individual patients developing clinical CMV infection after solid organ transplantation. An increase in s-creatinine levels, fever and poor allograft function are typically due to either rejection or CMV infection. It is, therefore of interest that the distribution of subsets within the CD4+ cell population, as reflected by the CD45RA:CD45RO ratio, was different between patients with rejection and those having CMV infection. In the former group, only one patient had the CMV type of CD45RA:CD45RO ratio. In that case, the histopathological picture in kidney biopsy was uncertain as to rejection. When individual cases were monitored prospectively, a switch in the CD45RA:CD45RO ratio heralded CMV infection, as confirmed by conventional criteria. Taken together, in renal allograft recipients showing elevated s-creatinine levels an analysis of the distribution of CD45RA⁺ vs. CD45RO⁺ cells within the CD4⁺ population is valuable in the differential diagnostics between rejection and clinically significant CMV infection. This finding is of importance with regard to treatment, since further immunosuppression (=anti-rejection treatment) in patients with CMV infection carries considerable morbidity and mortality.

Uremic patients on dialysis treatment had a CD45RA:CD45RO cell ratio similar to that found in transplanted cases with rejection but totally different from the ratio seen in CMV infected subjects. This finding further strengthens the diagnostic power of the CD45RA:CD45RO ratio. Further, the average cell ratio in transplanted patients with stable grafts was clearly different from that in patients with CMV infection.

All publications and patent applications mentioned in this specification are indicative of the level of ordinary skill in the art to which this invention pertains. All publications and patent applications are herein incorporated by reference to the same extent as if each individual publication or patent application was specifically and individually indicated to be incorporated by reference.

## Claims

1. A method for the prediction and detection of CMV infection in solid organ transplant patients comprising the steps of combining a sample of blood or other tissue from the patient with at least two fluorescently labelled monoclonal antibodies which recognize the CD45RO and CD45RA antigens on CD4⁺ T cells, counting on a flow cytometer the number of CD4⁺ T cells that are CD45RA⁺ and CD45RO⁺ and determining the ratio of such cells.

2. The method of claim 1 wherein the sample is combined with an anti-CD4 fluorescently labelled monoclonal antibody.

3. A method for the prediction and detection of CMV infection in solid organ transplant patients comprising the steps of combining a sample of blood or other tissue from the patient with at least three fluorescently labelled monoclonal antibodies which recognize the CD45RO, CD45RA and CD4 antigens on T cells, counting on a flow cytometer the number of CD4⁺ T cells that are CD45RA⁺ and CD45RO⁺ and determining the ratio of such cells.

4. The method of claim 1, 2 or 3 wherein the sample is further combined with an anti-HLA-DR fluorescently labelled monoclonal antibody.

5. The method of claim 1, 2, 3 or 4 wherein the antibodies are labelled with fluorescent dyes comprising the group consisting of peridinin chlorophyll complex, fluorescein isothiocyanate, phycoerthrin, Texas Red, allophycocyanin and tandem conjugates thereof.

6. The method of claim 1 or 3 wherein the step of counting cells is carried out by means of flow cytometry.

7. The use of at least two fluorescently labelled monoclonal antibodies which recognise the CD45RO and CD45RA antigens in the preparation of a kit for use in a method for the predication and detection of CMV infection in solid organ transplant patients, the method comprising the steps of combining a sample of blood or other tissue from the patient with at least two fluorescently labelled monoclonal antibodies which recognise the CD45RO and CD45RA antigens on CD4⁺ T cells, counting on a flow cytometer the number of CD4⁺T cells that are CD45RA⁺ and CD45RO⁺ and determining the ratio of such cells.

8. The use of at least three fluorescently labelled monoclonal antibodies which recognise the CD45RO, CD45RA, and CD4 antigens in the preparation of a kit for use in a method for the prediction and detection of CMV infection in solid organ transplant patients, the method comprising the steps of combining a sample of blood or other tissue from the patient with at least three fluorescently labelled monoclonal antibodies which recognise the CD45RO, CD45RA and CD4 antigens on T cells, counting on a flow cytometer number of CD4⁺T cells that are CD45RA⁺ and CD45RO⁺ and determining the ratio of such cells.

## Patentansprüche

1. Verfahren für die Vorhersage und den Nachweis von CMV Infektion in Festorgantransplantatpatienten umfassend die Stufen des Kombinierens einer Probe von Blut oder anderem Gewebe von dem Patienten mit mindestens zwei fluoreszierend markierten monoklonalen Antikörpern, welche die CD45RO und CD45RA Antigene auf CD4⁺ T Zellen erkennen, Zählen der Zahl von CD4⁺ T Zellen, die CD45RA⁺ und CD45RO⁺ sind, auf einem Fließzytometer und Bestimmen des Anteils derartiger Zellen.

2. Verfahren nach Anspruch 1, wobei die Probe mit einem Anti-CD4 fluoreszierend markierten monoklonalen Antikörper kombiniert wird.

3. Verfahren für die Vorhersage und den Nachweis von CMV Infektion in Festorgantransplantatpatienten, umfassend die Stufen des Kombinierens einer Probe von Blut oder anderem Gewebe von dem Patienten mit mindestens drei fluoreszierend markierten monoklonalen Antikörpern, welche die CD45RO, CD45RA und CD4 Antigene auf T Zellen erkennen, Zählen der Zahl von CD4⁺ T Zellen, die CD45RA⁺ und CD45RO⁺ sind, auf einem Fließzytometer und Bestimmen des Anteils derartiger Zellen.

4. Verfahren nach Anspruch 1, 2 oder 3, wobei die Probe ferner mit einem Anti-HLA-DR fluoreszierend markierten monoklonalen Antikörper kombiniert wird.

5. Verfahren nach Anspruch 1, 2, 3 oder 4, wobei die Antikörper mit Fluoreszenzfarbstoffen markiert werden, welche die Gruppe bestehend aus Peridininchlorophyllkomplex, Fluoresceinisothiocyanat, Phycoerythrin, Texas Red, Allophycocyanin und Tandemkonjugate davon umfassen.

6. Verfahren nach Anspruch 1 oder 3, wobei die Stufe des Zählens von Zellen mithilfe von Fließzytometrie durchgeführt wird.

7. Verwendung von mindestens zwei fluoreszierend markierten monoklonalen Antikörpern, welche die CD45RO und CD45RA Antigene erkennen, bei der Herstellung einer Ausrüstung (Kits) für die Verwendung in einem Verfahren für die Vorhersage und den Nachweis von CMV Infektion in Festorgantransplantatpatienten, wobei das Verfahren die Stufen des Kombinierens einer Probe von Blut oder anderem Gewebe von dem Patienten mit mindestens zwei fluoreszierend markierten monoklonalen Antikörpern, welche die CD45RO und CD45RA Antigene auf CD4⁺ T Zellen erkennen, Zählen der Zahl von CD4⁺ T Zellen, die CD45RA⁺ und CD45RO⁺ sind, auf einem Fließzytometer und Bestimmen des Anteils derartiger Zellen umfaßt.

8. Verwendung von mindestens drei fluoreszierend markierten monoklonalen Antikörpern, welche die CD45RO, CD45RA und CD4 Antigene erkennen, bei der Herstellung einer Ausrüstung (Kits) für die Verwendung in einem Verfahren für die Vorhersage und den Nachweis von CMV Infektion in Festorgantransplantatpatienten, wobei das Verfahren die Stufen des Kombinierens einer Probe von Blut oder anderem Gewebe von dem Patienten mit mindestens drei fluoreszierend markierten monoklonalen Antikörpern, welche die CD45RO, CD45RA und CD4 Antigene auf T Zellen erkennen, Zählen der Zahl von CD4⁺ T Zellen, die CD45RA⁺ und CD45RO⁺ sind, auf einem Fließzytometer und Bestimmen des Anteils derartiger Zellen umfaßt.

## Revendications

1. Procédé de prédiction et de détection de l'infection CMV chez des patients avec une greffe d'organe solide, comprenant les étapes de combinaison d'un échantillon de sang ou d'un autre tissu provenant d'un patient à au moins deux anticorps monoclonaux marqués par fluorescence reconnaissant les antigènes CD45RO et CD45RA sur les cellules CD4⁺ T, de comptage dans un cytomètre de flux du nombre de cellules CD4⁺ T qui sont CD45RA⁺ et CD45RO⁺, et de détermination du rapport de telles cellules.

2. Procédé de la revendication 1, dans lequel l'échantillon est combiné à un anticorps monoclonal anti-CD4 marqué par fluorescence.

3. Procédé de prédiction et de détection de l'infection CMV chez des patients avec une greffe d'organe solide, comprenant les étapes de combinaison d'un échantillon de sang ou d'un autre tissu provenant d'un patient à au moins trois anticorps monoclonaux marqués par fluorescence reconnaissant les antigènes CD45RO, CD45RA et CD4 sur les cellules T, de comptage dans un cytomètre de flux du nombre de cellules CD4⁺ T qui sont CD45RA⁺ et CD45RO⁺, et de détermination du rapport de telles cellules.

4. Procédé de la revendication 1, 2 ou 3, dans lequel l'échantillon est en outre combiné à un anticorps monoclonal anti-HLA-DR marqué par fluorescence.

5. Procédé de la revendication 1, 2, 3 ou 4, dans lequel les anticorps sont marqués avec des colorants fluorescents, comprenant le groupe composé d'un complexe de péridinine de chlorophylle, de l'isothiocyanate de fluorescéine, de la phycoérythrine, du Rouge Texas, de l'allophycocyanine ainsi que de conjugués tandem de ceux-ci.

6. Procédé de la revendication 1 ou 3, dans lequel l'étape de comptage de cellules est réalisée par cytométrie de flux.

7. Utilisation d'au moins deux anticorps monoclonaux marqués par fluorescence reconnaissant les antigènes CD45RO et CD45RA dans la préparation d'un kit destiné à l'utilisation dans un procédé de prédiction et de détection de l'infection CMV chez des patients avec une greffe d'organe solide, le procédé comprenant les étapes de combinaison d'un échantillon de sang ou d'un autre tissu provenant d'un patient à au moins deux anticorps monoclonaux marqués par fluorescence reconnaissant les antigènes CD45RO et CD45RA sur les cellules CD4⁺ T, de comptage dans un cytomètre de flux du nombre de cellules CD4⁺ T qui sont CD45RA⁺ et CD45RO⁺, et de détermination du rapport de telles cellules.

8. Utilisation d'au moins trois anticorps monoclonaux marqués par fluorescence reconnaissant les antigènes CD45RO, CD45RA et CD4 dans la préparation d'un kit destiné à l'utilisation dans un procédé de prédiction et de détection de l'infection CMV chez des patients avec une greffe d'organe solide, le procédé comprenant les étapes de combinaison d'un échantillon de sang ou d'un autre tissu provenant d'un patient à au moins trois anticorps monoclonaux marqués par fluorescence reconnaissant les antigènes CD45RO, CD45RA et CD4 sur les cellules T, de comptage dans un cytomètre de flux du nombre de cellules CD4⁺ T qui sont CD45RA⁺ et CD45RO⁺, et de détermination du rapport de telles cellules.
